# EUROPEAN PATENT APPLICATION

(11) **EP 0 933 332 A1**
(43) Date of publication of application: **04.08.1999**
(21) Application number: 99300565.1
(22) Date of filing: 26.01.1999
(51) Int. Cl.: C02F 1/30, C02F 1/46, A61K 33/00, A61K 35/08

(54) **Electrolysis of water resulting in the reduction of fat content in the human liver**

(30) Priority: 26.01.1998 US 13110
(71) Applicant: Kim, Hee Jung, Seoul 138-220 (KR); Shin, Myoung Soo, Seoul 136-020 (KR)
(72) Inventor: Kim, Hee Jung, Songpa-ku, Seoul 138-220 (KR); Shin, Myoung Soo, Seoul 136-020 (KR); Park, Sang Chul, Seocho-ku, Seoul 137-044 (KR)
(74) Representative: Evens, Paul Jonathan

(57) **Abstract**

A process for producing water that reduces the fat content of a human liver when consumed. This water is the product of a multi-step process comprising a plurality of separate batch processes, wherein each successive process yields a product with a higher affinity to fat than the product yielded from the previous process. The first process comprises the passage of an electric current through water without the presence of electrolytes, which yields water with a higher affinity to fat. The resulting water is then placed into a series of batch energiser reactors, wherein the water is successively irradiated with far infrared light in each batch reactor for a specified resident time period, each successive reactor yielding a product with a higher affinity to fat. Receivers at each batch reactor allow the water to be properly drained from the reactors. Water with a higher affinity to fat retains the fat and prevents it from absorbing into the liver.

## Description

### BACKGROUND OF THE INVENTION

### 1. Field of the Invention

The present invention relates generally to chemical processes and pharmaceuticals used in the reduction of fat in the liver.

### 2. Description of the Prior Art

It is commonly known that excess fat in the liver may lead to serious health problems. Many remedies have been proposed in solving this dilemma.

The prior art pertaining to body fat reduction include the following. U.S. Patent No. 4,865,850 to Shell et al. discloses a method and a composition for accumulating and the binding of food fats in an animal body gastrointestinal tract U.S. Patent No. 5,610,152 to Suzuki discloses an anti-obesity agent comprising 4-cholesten-3-one as an effective component. U.S. Patent 5,597,797 to Clark discloses a method for treating obese mammals or preventing obesity from occurring in mammals.

Although the prior art teach inventions pertaining to the reduction of body fat, they do not teach a process specifically reducing the fat content of a human liver. Hence, this process in novel and unanticipated.

Consequently, the primary object of the present invention is to provide a pharmaceutical product used to reduce the amount of fat in the liver through oral ingestion of the product.

### SUMMARY OF THE INVENTION

Electrolysis of water yields a solution wherein hydrogen bonding between water molecules is lessened. This results in a solution with smaller water molecule clusters, since water molecule clusters are formed due to hydrogen bonding between water molecules. Because there are less water molecules in each cluster, the net charge of a given cluster has a greater probability of having a stronger charge than that of a larger cluster, since there is more of a chance that molecules may be clustered in an orientation yielding a larger charge.

Therefore, the resulting water has been found to have a higher polarity than ordinary tap water, thus allowing it to dissolve fat molecules. Fat molecules with induced positive and negative dipoles have a higher affinity for water. Thus, a greater number of fat molecules remain in the blood stream and are subsequently burned. Fat molecules are thus burned, eventually lowering the level of fat in the liver.

In laboratory tests, this water was found to reduce the fat level in rat livers by thirty-five percent.

These together with other objects of the invention are explained clearly in the claims annexed to and forming a part of this disclosure. For a better understanding of the invention, its operating advantages and the specific objects attained by its use, reference should be made to the accompanying drawings and descriptive matter in which there are illustrated preferred embodiments of the invention.

### BRIEF DESCRIPTION OF THE DRAWINGS

For a better understanding of the principle and nature of the present invention, reference should be made to the following detailed description taken in connection with the accompanying drawings in which:

**FIG. 1** is a block flow diagram of the process for reducing the viscosity of water.

**FIG. 2a** is a graph of LDH (U/ g liver) vs. time (minutes).

**FIG. 2b** is a graph of MDA (nmol/mg protein) vs. Groups.

**FIG. 3** is a graph of ethanol residual in vascular perfusate (% of infused amount).

### DETAILED DESCRIPTION OF THE PREFERRED EMBODIMENT

The present invention is a multi-step process that comprises a plurality of batch processes, wherein the product yielded from a step in the overall process is then successively used as the feed for the subsequent batch process, yielding a product useful in lessening the fat content of a human liver.

Electrolysis of water yields a solution wherein hydrogen, bonding between water molecules is lessened. This results in a solution with smaller water molecule clusters, since water molecule clusters are formed due to hydrogen bonding between water molecules. Because there are less water molecules in each cluster, the net charge of a given cluster has a greater probability of having a stronger charge than that of a larger cluster, since there is more of a chance that molecules may be clustered in an orientation yielding a larger charge.

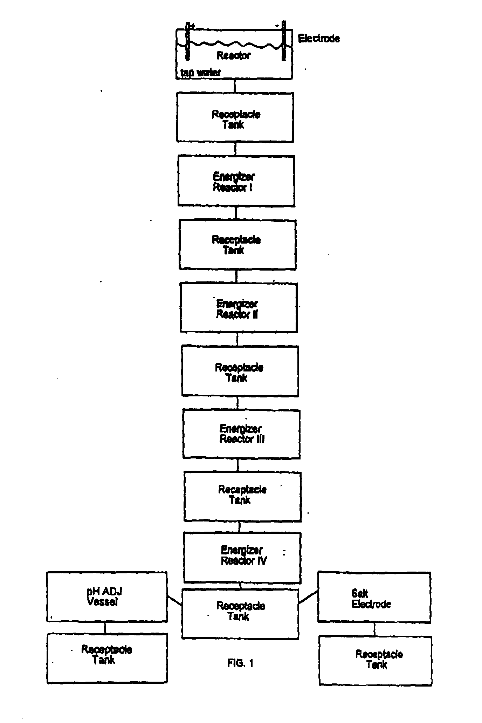

Referring to FIG. 1, the first batch process involves the passage of an electrical current through water, The water used is preferably filtered and is without electrolytes or contaminants, so as to maximize the oxygen content within the water, as water with dissolved contaminants like salt is unable to be fully saturated with oxygen. The voltage of the current is preferably between 50 and 100 volts. The process may use multiple cathodic and anodic leads, so as to maximize the effects of the current. In addition, stirrers may be employed in the reaction as well. In the present invention, the amount of dissociation of the aforementioned water clusters is proportional to the amount of time the current is passed through the water.

The second batch process comprises an energizing reactor that comprises a batch process tank reactor with several far infrared light emitters. These emitters are positioned along the tank so as to maximize exposure to the water. The effluent yielded from the first batch process is then fed into the energizer reactor where the water is irradiated with far infrared light by the emitters.

The resulting water has been found to have a higher polarity than ordinary tap water, thus allowing it to dissolve fat molecules. Fat molecules with induced positive and negative dipoles have a higher affinity for water. Thus, a greater number of fat molecules remain in the blood steam and are subsequently burned. The presently invented water also has smaller water molecule clusters than ordinary tap water, rendering a higher rate of delivery to blood vessels. Fat molecules which are thus burned, eventually lowering the level of fat in the liver.

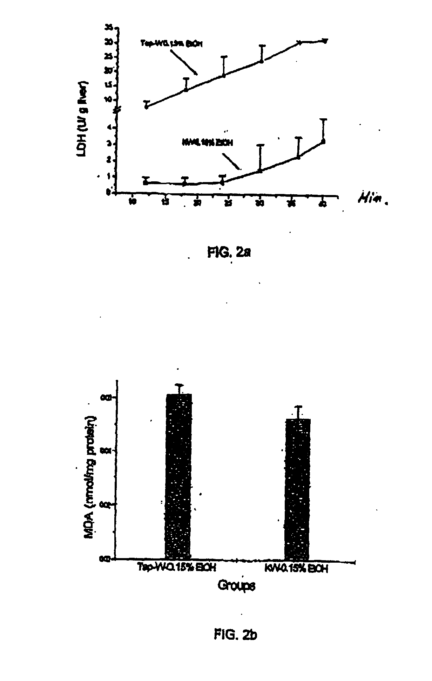

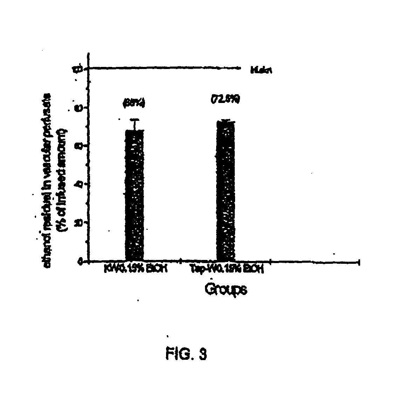

Referring now to **FIGS. 2a, 2b and 3**, the results of laboratory tests are shown in the form of comparative graphs. It should be noted that the presently invented water has been found to reduce the fat level in rat livers substantially over tap water.

## Claims

1. A process for producing water with a greater affinity to fat comprising:
a) filtration of water;
b) electrolysis of said water using a cathode and anode, and a current of voltage ranging between 50 to 100 volts;
c) irradiation of said water by far infrared light

2. A process for producing water with a greater affinity to fat as mentioned in claim 1, wherein said water is irradiated by far infrared light of varying wavelengths.

3. A process for producing water with a greater affinity to fat as mentioned in claim 1, wherein said water is irradiated by said far infrared light for a period ranging between four and twelve hours.

4. A process for producing water with a greater affinity to fat as mentioned in claim 1, wherein pH of said water is adjusted.

5. A process for producing water with a greater affinity to fat as mentioned in claim 1, wherein said water is introduced to a salt electrode.

6. The water of claims 1-5 resulting in the reduction of the fat content of a human liver.

7. A pharmaceutical composition for reducing liver fat comprising water processed to provide smaller clusters of water molecules of higher polarity than tap water.
